(19) Europäisches Patentamt
European Patent Office
Office européen des brevets

(11) Veröffentlichungsnummer: **0 127 864**
**A2**

# (12) EUROPÄISCHE PATENTANMELDUNG

(21) Anmeldenummer: **84106046.0**

(22) Anmeldetag: **28.05.84**

(51) Int. Cl.³: **C 07 J 63/00**
**A 61 K 31/13**

(30) Priorität: **03.06.83 DE 3320580**

(43) Veröffentlichungstag der Anmeldung:
**12.12.84 Patentblatt 84/50**

(84) Benannte Vertragsstaaten:
**AT BE CH DE FR GB IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65(DE)**

(72) Erfinder: **Neef, Günter, Dr.**
**Darmstädter Strasse 9**
**D-1000 Berlin 15(DE)**

(72) Erfinder: **Sauer, Gerhard, Dr.**
**Königsbacher Zeile 47 A**
**D-1000 Berlin 28(DE)**

(72) Erfinder: **Wiechert, Rudolf, Prof. Dr.**
**Petzower Strasse 8 A**
**D-1000 Berlin 39(DE)**

(72) Erfinder: **Beier, Sybille, Dr.**
**Uhlandstrasse 121**
**D-1000 Berlin 31(DE)**

(72) Erfinder: **Elger, Walter, Dr.**
**Schorlemer Allee 12 B**
**D-1000 Berlin 33(DE)**

(72) Erfinder: **Henderson, David, Dr.**
**Jahnstrasse 17**
**D-1000 Berlin 28(DE)**

(54) **D-Homo-4,9,16-estratriene, deren Herstellung und diese enthaltende pharmazeutische Präparate.**

(57) Es werden D-Homo-4,9,16-estratriene der allgemeinen Formel I

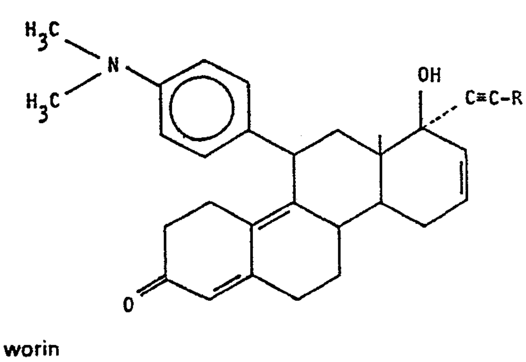

(I),

worin

R ein Wasserstoffatom, ein Chloratom oder eine Methylgruppe

bedeuten,

beschrieben, die starke antigestagene Wirkung besitzen und zur postcoitalen Fertilitätskontrolle verwendet werden können.

Die Erfindung betrifft D-Homo-4,9,16-estratriene der allgemeinen Formel I, ein Verfahren zu deren Herstellung und diese enthaltende pharmazeutische Präparate.

Die neuen Verbindungen der allgemeinen Formel I sind gekennzeichnet durch einen substituierten Phenylring in 11-Stellung des Steroidgerüstes. Solche Verbindungen werden beschrieben zum Beispiel in Steroids 37 (1981) Seite 361 ff. und in der europäischen Patentanmeldung 82400025.1 (Veröffentlichungsnummer 0 057 115). Die D-Homo-Verbindungen der allgemeinen Formel I werden auch von dem allgemeinen Anspruch der europäischen Patentanmeldung umfaßt, es werden jedoch keine D-Homo-Verbindungen in der europäischen Patentanmeldung beschrieben.

Es wurde gefunden, daß die neuen D-Homo-Verbindungen der allgemeinen Formel I eine außergewöhnlich starke antigestagene Wirkung besitzen.

Zur Kennzeichnung der antigestagenen Wirkung wurde die abortive Wirkung bestimmt.

Die Versuche wurden an weiblichen Ratten im Gewicht von ca. 200 g durchgeführt. Nach erfolgter Anpaarung wurde der Schwangerschaftsbeginn durch Nachweis von Spermien in Vaginalabstrichen gesichert. Der Tag des Spermiennachweises gilt als Tag 1 der Gravidität (= d1 p.c.).

Die Behandlung der Tiere mit der jeweils zu testenden Substanz bzw. dem Lösungsmittel erfolgte nach der Nidation der Blastocysten von d5 p.c. bis d7 p.c.

An d9 p.c. wurden die Tiere getötet und die Uteri auf Implantate und Resorptionsstellen hin untersucht. Von allen Uteri wurden Fotos angefertigt. Das Fehlen von Implantaten wurde als Abort gewertet.

Die Testsubstanzen wurden in einem Benzylbenzoat-Rizinusöl-Gemisch (Verhältnis 1 + 9) gelöst. Das Vehikelvolumen pro Einzeldosis betrug 0,2 ml. Die Behandlung erfolgte subcutan.

Testsubstanzen waren die erfindungsgemäße Verbindung A und das in der europäischen Patentanmeldung 82 400 025.1 beschriebene 11ß-(4-Dimethylaminophenyl)-17ß-hydroxy-17α-(propin-1-yl)-4,9(10)-estradien-3-on (B).

<u>Antigestagene Wirkung (Abortauslösung Ratte)</u>

| | Dosis mg/Tier/Tag | n Abort / n Gesamt |
|---|---|---|
| 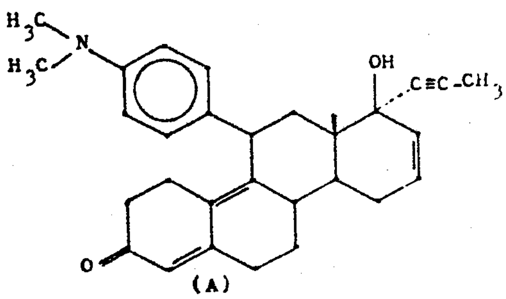 (A) | 10.0 | 4/4 |
| | 3.0 | 4/4 |
| | 1.0 | 4/4 |
| | 0.3 | 2/4 |
| 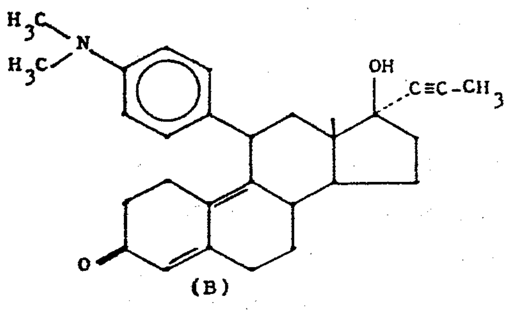 (B) | 10.0 | 4/4 |
| | 3.0 | 4/4 |
| | 1.0 | 2/4 |

Die Tabelle zeigt, daß die erfindungsgemäße Verbindung noch in einer Dosierung von 1,0 mg voll wirksam ist, während mit der Vergleichsverbindung nur noch zu 50 % Aborte ausgelöst werden können.

Aufgrund der starken antigestagenen Wirkung können die neuen D-Homo-Verbindungen der allgemeinen Formel I bei der postcoitalen Fertilitätskontrolle Verwendung finden.

Die Erfindung betrifft auch pharmazeutische Präparate, die D-Homo-Verbindungen der allgemeinen Formel I enthalten. Die Herstellung der Präparate erfolgt nach an sich bekannten Methoden der Galenik durch Mischen mit einem für die enterale, perkutane oder parenterale

Applikation geeigneten pharmazeutischen, organischen oder anorganischen inerten Trägermaterial.

Die Dosierung der erfindungsgemäßen Wirkstoffe liegt beim Menschen bei 10 bis 1000 mg pro Tag.

Die neuen Verbindungen der Formel I werden erfindungsgemäß nach dem Verfahren gemäß Ansoruch 6 hergestellt. Das erfindungsgemäße Verfahren wird in einem wasserfreien aprotischen Lösungsmittel wie Tetrahydrofuran (THF), Dioxan oder Diethyläther durchgeführt, indem man Magnesiumspäne mit dem Arylhalogenid 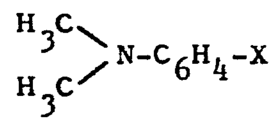

(mit X in der oben angegebenen Bedeutung), falls erforderlich in Gegenwart von Methyliodid als Katalysator, bei 20 °C bis 50 °C, vorzugsweise bis 45 °C, zur Grignard-Verbindung umsetzt. Nach beendeter Reaktion setzt man der erhaltenen Grignard-Lösung bei 0 °C bis 10 °C, vorzugsweise bei 0 °C bis 5 °C, die entsprechende Menge Kupfer-I-chlorid zu, rührt die Reaktionsmischung 10 bis 20 Minuten unter Eiskühlung und gibt die Lösung der Ausgangsverbindung der allgemeinen Formel II, gelöst in einem geeigneten wasserfreien aprotischen Lösungsmittel wie Tetrahydrofuran (THF), Dioxan oder Diethyläther, vorzugsweise THF, zu und rührt 1 bis 20 Stunden, vorzugsweise 1 bis 5 Stunden, bei 20 °C und arbeitet die Reaktionsmischung auf. Der genaue Zeitpunkt des Reaktionsendes wird vorzugsweise durch Dünnschichtchromatographie bestimmt.

Das so erhaltene Rohprodukt, das die 3-Ketalgruppe und eine 5α-Hydroxygruppe enthält, wird nach bekannten Verfahren in einem mit Wasser mischbaren Lösungsmittel wie wäßrigem Ethanol oder Methanol oder Aceton gelost und die

Lösung in Gegenwart von katalytischen Mengen Mineralsäure oder Sulfonsäure wie Salzsäure, Schwefelsäure, Perchlorsäure oder p-Toluolsulfonsäure so lange zum Sieden erhitzt, bis die Ketalspaltung und Wasserabspaltung beendet sind, was durch Dünnschichtchromatographie ermittelt wird. Nach Aufarbeitung und Reinigung erhält man die erfindungsgemäßen Verbindungen der allgemeinen Formel I.

Beispiel 1

11ß-(4-Dimethylaminophenyl)-17aß-hydroxy-17aα-(1-propinyl)-D-homo-4,9,16-estratrien-3-on

a) Eine Lösung von 3,55 g D-Homo-4,9,16-estratrien-3,17a-dion in 35 ml Methylenchlorid wird sukzessive mit 3,55 g 2,2-Dimethyl-propan-1,3-diol, 1,75 ml Orthoameisensäuretrimethylester und 20 mg p-Toluolsulfonsäure versetzt. Man rührt 2,5 Stunden bei Raumtemperatur, verdünnt anschließend mit 150 ml Methylenchlorid, wäscht mit gesättigter $NaHCO_3$-Lösung, trocknet über $Na_2SO_4$ und engt ein. Der ölige Rückstand wird über $Al_2O_3$ (neutral III) mit Hexan/Essigester chromatographiert. Nach Kristallisation der Hauptfraktion aus Diisopropylether erhält man 3,3 g 3,3-(2,2-Dimethyl-propan-1,3-dioxy)-D-homo-5(10),9(11),16-estratrien-17a-on vom Schmelzpunkt 154 - 156 °C.

b) Zu einer Lösung von 2,5 g 3,3-(2,2-Dimethyl-propan-1,3-dioxy)-D-homo-5(10),9(11),16-estratrien-17a-on in 14 ml Methylenchlorid, 0,11 ml Hexachloraceton und 0,01 ml Pyridin tropft man unter Eiswasserkühlung 1,32 ml 30 %ige $H_2O_2$. Man rührt 16 Stunden bei Raumtemperatur, verdünnt mit 100 ml Methylenchlorid, wäscht mit 5 %iger $Na_2S_2O_3$-Lösung und engt ein. Das so erhaltene Gemisch aus 5α,10α-Epoxid und 5ß,10ß-Epoxid wird durch Chromatographie über $Al_2O_3$ mit Hexan/Essigester getrennt. Man erhält nach Kristallisation aus Essigester/Diisopropylether 1,46 g 3,3-(2,2-Dimethyl-propan-1,3-dioxy)-5α,10α-epoxy-D-homo-9(11),16-estradien-17a-on vom Schmelzpunkt 188 - 191 °C.

0127864

c) 130 ml absolutes THF werden bei +5 °C mit Methylacetylen gesättigt. Anschließend tropft man unter Eiswasserkühlung 27 ml einer 15 %igen Lösung von n-Butyllithium in Hexan hinzu und danach eine Lösung von 1,43 g 3,3-(2,2-Dimethyl-propan-1,3-dioxy)-5α,10α-epoxy-D-homo-9(11),16-estradien-17a-on in 20 ml absolutem THF. Man rührt 3 Stunden bei 25 °C, gießt in Eiswasser und extrahiert mit Essigester. Das so erhaltene Rohprodukt (1,6 g) wird ohne weitere Reinigung in die Folgestufe eingesetzt.

d) Zu einer Suspension aus 688 mg Magnesiumspänen in 10 ml absolutem THF gibt man zunächst 0,005 ml Methyliodid und anschließend eine Lösung von 6,6 g 4-Bromdimethylanilin in 32 ml THF so hinzu, daß die Innentemperatur 45 °C nicht übersteigt. Nach Auflösung des Magnesiums kühlt man auf 0 °C, gibt 146 mg wasserfreies Kupfer(I)chlorid hinzu und rührt 15 Minuten bei 0 °C, bevor eine Lösung von 1,6 g des unter c) erhaltenen Produkts /3,3-(2,2-Dimethyl-propan-1,3-dioxy)-5α,10α-epoxy-17aα-(1-propinyl)-D-homo-9(11),16-estradien-17aß-ol7 in 13 ml THF hinzugetopft wird. Nach Zugabe rührt man noch 1,5 Stunden bei Raumtemperatur, gießt in Eiswasser, filtriert über Celite und extrahiert das Filtrat mit Essigester. Die Essigester-Extrakte werden getrocknet ($Na_2SO_4$) und im Vakuum eingeengt. Das so erhaltene Rohprodukt wird in 15 ml 70 %iger Essigsäure 30 Minuten bei 60 °C gerührt. Danach gießt man in Eiswasser, stellt durch Zugabe von konzentrierter $NH_3$-Lösung einen pH-Wert von 10,5 ein und extrahiert erneut mit Essigester. Nach Chromatographie des Rohprodukts über $Al_2O_3$ mit Hexan/Essigester erhält man 1,1 g 11ß-(4-Dimethylaminophenyl)-17aß-hydroxy-17aα-(1-propinyl)-D-homo-4,9,16-estratrien-3-on als farbloses Öl.

$^1$H-NMR ($CDCl_3$): δ = 0,59 ppm (s,3H,H-18); 1,89 (s,3H,C≡C-$CH_3$); 2,90 (s,6H,N-$CH_3$); 4,33 (m,1H,H-11); 5,57 (m,2H,H-16,H-17); 5,75 (s,1H,H-4); 6,63 u. 7,02 (AA'BB', je 2H, aromat. H).

## Beispiel 2

11ß-(4-Dimethylaminophenyl)-17aα-ethinyl-17aß-hydroxy-D-homo-4,9,16-estratrien-3-on

a) 170 ml absolutes THF werden unter Eiswasserkühlung mit Acetylen gesättigt. Anschließend tropft man 34 ml einer 15 %igen Lösung von n-Butyllithium in Hexan hinzu und rührt 15 Minuten bei +5 °C. Dann wird eine Lösung von 1,68 g 3,3-(2,2-Dimethyl-propan-1,3-dioxy)-5α,10α-epoxy-D-homo-9(11),16-estradien-17a-on in 24 ml THF tropfenweise hinzugegeben. Man rührt 60 Minuten bei Raumtemperatur und arbeitet wie unter Beispiel 1 c) auf. Nach Kristallisation aus Hexan/Ethanol erhält man 1,45 g 3,3-(2,2-Dimethyl-propan-1,3-dioxy)-5α,10α-epoxy-17aα-ethinyl-D-homo-9(11),16-estradien-17aß-ol vom Schmelzpunkt 192 - 196 °C.

b) Unter den Bedingungen des Beispiels 1 d) stellt man aus 732 mg Magnesium, 0,005 ml Methyliodid, 7,4 g 4-Bromdimethylanilin und 160 mg CuCl ein Grignard-Reagenz her. Die Umsetzung mit 1,7 g 3,3-(2,2-Dimethyl-propan-1,3-dioxy)-5α,10α-epoxy-17aα-ethinyl-D-homo-9(11),16-estradien-17aß-ol erfolgt analog Beispiel 1 d). Nach saurer Spaltung und Chromatographie erhält man 1,05 g 11ß-(4-Dimethylaminophenyl)-17aα-ethinyl-17aß-hydroxy-D-homo-4,9,16-estratrien-3-on vom Schmelzpunkt 152 - 154 °C.
$^1$H-NMR ($CDCl_3$): δ = 0,58 ppm (s,3H,H-18); 2,61 (s,1H,C≡CH); 2,90 (s,6H,$NCH_3$); 4,34 (m,1H,H-11); 5,60 (m,2H,H-16,H-17); 5,76 (s,1H,H-4); 6,60 u. 7,03 (AA'BB', je 2 H, aromat. H).

Beispiel 3

17aα-Chlorethinyl-11ß-(4-dimethylaminophenyl)-17aß-hydroxy-D-homo-4,9,16-estratrien-3-on

Lithium-chloracetylid wird in situ aus cis-Dichlorethylen und Methyllithium in Ether hergestellt /H.G. Viehe, Chem. Ber. 92, 1950 (1959)/ und an 3,3-(2,2-Dimethyl-propan-1,3-dioxy)-5α,10α-epoxy-D-homo-9(11),16-estradien-17a-on addiert. Nach Grignard-Reaktion und saurer Spaltung analog Beispiel 1 erhält man 17aα-Chlorethinyl-11ß-(4-dimethylaminophenyl)-17aß-hydroxy-D-homo-4,9,16-estratrien-3-on vom Schmelzpunkt 168 - 172 °C.

0127864

Patentansprüche für die Vertragsstaaten BE, CH, DE, FR, GB, IT, LU, NL, SE

1.) D-Homo-4,9,16-estratriene der allgemeinen Formel I

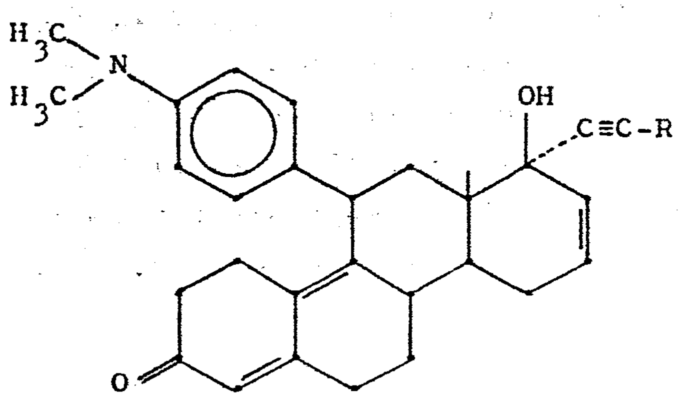

(I),

worin

R ein Wasserstoffatom, ein Chloratom oder eine Methylgruppe

bedeuten.

2.) 11ß-(4-Dimethylaminophenyl)-17aß-hydroxy-17aα-(1-propinyl)-D-homo-4,9,16-estratrien-3-on.

3.) 11ß-(4-Dimethylaminophenyl)-17aα-ethinyl-17aß-hydroxy-D-homo-4,9,16-estratrien-3-on.

4.) 17aα-Chlorethinyl-11ß-(4-dimethylaminophenyl)-17aß-hydroxy-D-homo-4,9,16-estratrien-3-on.

5.) Pharmazeutische Präparate, gekennzeichnet durch den Gehalt an einer Verbindung gemäß Anspruch 1 bis 4.

6.) Verfahren zur Herstellung von D-Homo-4,9,16-estratrienen der allgemeinen Formel I

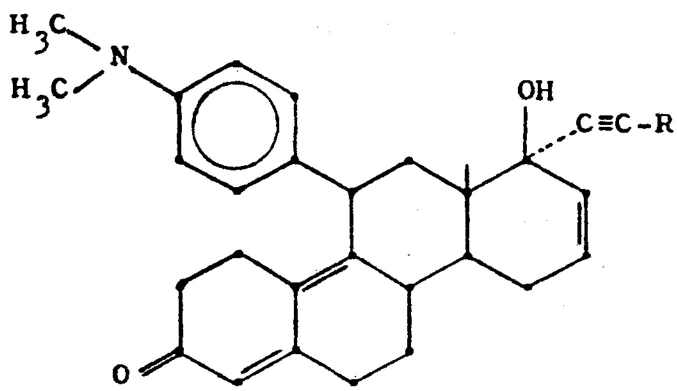

(I),

worin

R ein Wasserstoffatom, ein Chloratom oder eine Methylgruppe

bedeuten,

dadurch gekennzeichnet, daß man in an sich bekannter Weise eine Verbindung der allgemeinen Formel II

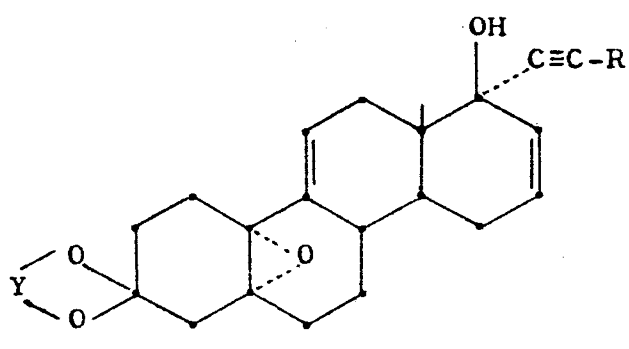

(II),

worin

R die in Formel I angegebene Bedeutung hat und

Y eine Ethylen- oder 2,2-Dimethylpropylengruppe

darstellt
mit einer Grignard-Verbindung der allgemeinen Formel III

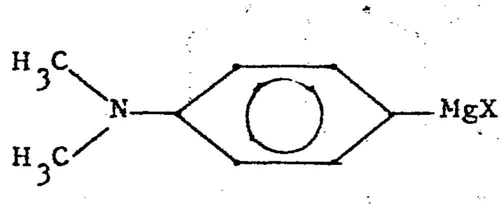

(III),

worin X ein Chlor- oder Bromatom darstellt, in Gegenwart von CuCl umsetzt und anschließend zur Spaltung des 3-Ketalschutzes und zur Wasserabspaltung aus 4,5-Stellung unter Ausbildung der 4,5-Doppelbindung mit Säure behandelt.

0127864

Patentanspruch für den Vertragsstaat AT

Verfahren zur Herstellung von D-Homo-4,9,16-estratrienen der allgemeinen Formel I

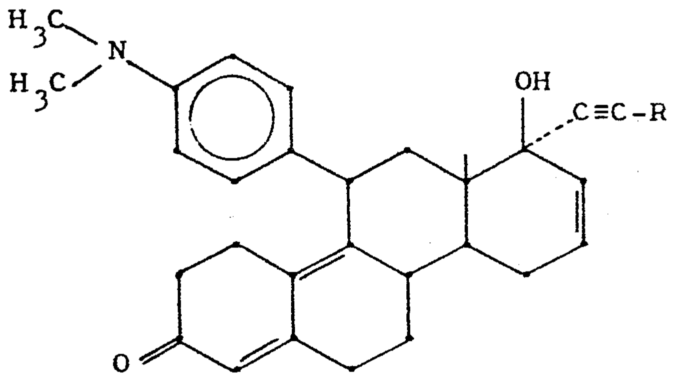

(I),

worin

R ein Wasserstoffatom, ein Chloratom oder eine Methylgruppe

bedeuten,

dadurch gekennzeichnet, daß man in an sich bekannter Weise eine Verbindung der allgemeinen Formel II

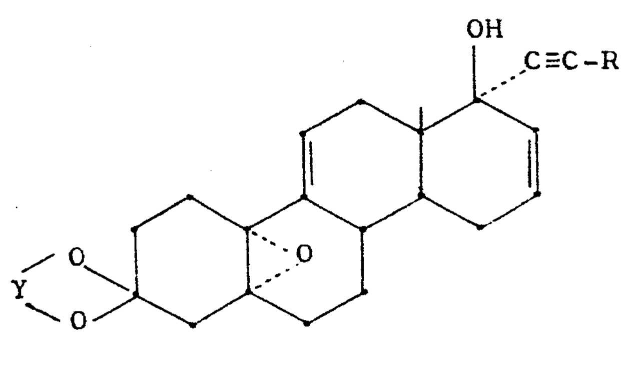

(II),

worin

R die in Formel I angegebene Bedeutung hat und

Y eine Ethylen- oder 2,2-Dimethylpropylengruppe

darstellt
mit einer Grignard-Verbindung der allgemeinen Formel III

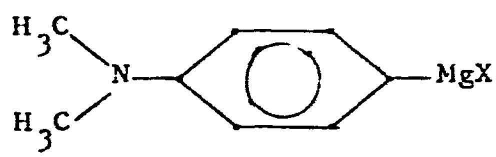

(III),

worin X ein Chlor- oder Bromatom darstellt, in Gegenwart von CuCl umsetzt und anschließend zur Spaltung des 3-Ketalschutzes und zur Wasserabspaltung aus 4,5-Stellung unter Ausbildung der 4,5-Doppelbindung mit Säure behandelt.